Europäisches Patentamt

European Patent Office

Office européen des brevets

(19)

(11) Veröffentlichungsnummer: **0 175 079 B1**

(12) **EUROPÄISCHE PATENTSCHRIFT**

(45) Veröffentlichungstag der Patentschrift:
**07.01.88**

(21) Anmeldenummer: **85108673.6**

(22) Anmeldetag: **11.07.85**

(51) Int. Cl.⁴: **A 61 F 2/36**, A 61 F 2/46

(54) Gelenkendoprothese und Instrument zum Ein- bzw. Ausschlagen derselben.

(30) Priorität: **14.09.84 DE 3433859**

(43) Veröffentlichungstag der Anmeldung:
**26.03.86 Patentblatt 86/13**

(45) Bekanntmachung des Hinweises auf die Patenterteilung:
**07.01.88 Patentblatt 88/1**

(84) Benannte Vertragsstaaten:
**CH DE FR GB IT LI NL SE**

(56) Entgegenhaltungen:
**EP - A - 0 027 159**
**CH - A - 459 462**
**DE - B - 2 101 002**
**DE - B - 2 134 316**
**DE - U - 8 400 642**
**US - A - 4 222 382**

(73) Patentinhaber: **Waldemar Link GmbH & Co,
Barkhausenweg 10, D-2000 Hamburg 63 (DE)**

(72) Erfinder: **Keller, Arnold, An der Naherfurth 5,
D-2061 Kayhude (DE)**

(74) Vertreter: **Glawe, Delfs, Moll & Partner Patentanwälte,
Postfach 26 01 62 Liebherrstrasse 20,
D-8000 München 26 (DE)**

ACTORUM AG

## Beschreibung

Die Erfindung bezieht sich auf eine Gelenkendoprothese und ein Instrument zum Ein- bzw. Ausschlagen derselben, die zur gegenseitigen Verbindung mit zusammenwirkenden Rippen und Nuten einerseits in einer am Ende des Instrumentenschafts angeordneten Gabel und andererseits am Hals der Prothese vorgesehen sind, wobei die Richtung des Instrumentenschafts quer zur Gabelebene verläuft.

Bei bekannten Prothesen- und Instrumentenkombinationen dieser Art weist der Prothesenhals an gegenüberliegenden Seiten je eine Nut auf, in die die Gabelzinken des Instruments eingreifen, wobei die gemeinsame Ebene der Gabelzinken derart geneigt zur Schaftrichtung verläuft, dass die Einschlagkräfte die Gabel in Eingriff mit dem Prothesenhals zwingen. Umgekehrt würden Ausschlagkräfte die Gabel vom Prothesenhals abziehen; die bekannte Kombination ist deshalb zum Ausschlagen der Prothese nicht geeignet. Dafür wird vielmehr ein besonderes Instrument vorgesehen, das mit einer Hakengabel unter den Gelenkkopf fasst. Ein solches Ausschlaginstrument ist jedoch nicht brauchbar bei Prothesen mit über eine Konusverbindung lösbaren Prothesenkopf, da in einem solchen Fall das Angreifen des Instruments am Kopf lediglich dessen Lösung vom Prothesenschaft bewirken würde. Ausserdem hat die bekannte Instrumenten/Prothesen-Kombination den Nachteil, dass selbst bei genauer Fertigung ein gewisses Spiel zwischen der Instrumentengabel und den Nuten am Prothesenhals nicht auszuschliessen ist, wodurch die viel Feingefühl verlangende Rotationseinstellung der Prothese erschwert sein kann.

Der Erfindung liegt die Aufgabe zugrunde, eine Gelenkendoprothese und ein dazu passendes Instrument zum Ein- bzw. Ausschlagen zu schaffen, die diese Nachteile nicht aufweisen.

Die erfindungsgemässe Lösung besteht darin, dass das Ende des Instrumentenschafts als Arretierstift durch eine Bohrung der Gabel hindurchragt und die Prothese eine Arretierausnehmung zur Aufnahme des Arretierstifts aufweist.

Zweckmässigerweise ist die Arretierungsausnehmung eine Bohrung; jedoch ist dies nicht unbedingt erforderlich, weil die Sicherung der Gabel an dem Prothesenhals auch durch den Eingriff des Arretierstifts in eine umlaufende Nut sichergestellt werden könnte. Wenn zur Aufnahme des Arretierstifts Einzelausnehmungen vorgesehen sind, nämlich beispielsweise Bohrungen, so sind zweckmässigerweise mehrere dieser Einzelausnehmungen über den Umfang oder den in Frage kommenden Umfangsbogen der Prothese verteilt, damit das Instrument in unterschiedlichen Stellungen mit der Prothese verbunden werden kann, angepasst an unterschiedliche Operationsbedingungen.

Nach einer Ausgestaltung der Erfindung sollen der Arretierstift und die Arretierausnehmung über wenigstens eine quer zur Schaftrichtung verlaufende Fläche zusammenwirken. Dadurch wird nämlich eine unmittelbare Schlagkraftübertragung von dem Instrumentenschaft auf die Prothese — unter Umgehung der Gabel — geschaffen, die es gestattet, die Passflächen an der Gabel und am Prothesenhals entsprechend feiner zu gestalten, da sie nur geringe Kräfte aufzunehmen brauchen. Auch die Gabel selbst und ihre Verbindung mit dem Schaft kann entsprechend feiner ausgeführt werden, was im Hinblick auf die beschränkten Platzverhältnisse im Operationsfeld von grosser Bedeutung sein kann.

Nach einer weiteren Ausgestaltung der Erfindung sind der Schaft und die ihn in der Gabel aufnehmende Bohrung mit zusammenwirkendem Gewinde versehen. Dies ermöglicht einerseits besondere Einfachheit in der Ausbildung der genannten Teile, weil keine zusätzlichen Verbindungsglieder benötigt werden, die Raum beanspruchen und störungsanfällig sind, und andererseits wird dadurch eine einfache, die Aufmerksamkeit des Arztes von wichtigeren Problemen nicht ablenkende Betätigungsmöglichkeit für die Verbindung zwischen Instrument und Prothese geschaffen, die einfach durch Drehen in der einen oder anderen Richtung geschlossen bzw. gelöst wird. Jedoch wäre es selbstverständlich auch möglich, eine andere Verbindungstechnik zwischen Schaft und Gabel zu wählen, die einerseits eine Längsbewegung des den Arretierstift bildenden Schaftendes bezüglich der Gabel gestattet und andererseits den Arretierstift in der Arretierstellung festhält, beispielsweise eine Bajonettverbindung.

Vorteilhaft kann es sein, die prothesenseitige Rippe bzw. Nut am Prothesenhals ringförmig auszubilden. Dadurch wird es nämlich dem Operateur gestattet, die Gabel des Instruments in einer anderen als der durch die Arretierung ursprünglich festgelegten Verbindungsrichtung von dem Prothesenhals abzuziehen, wenn beispielsweise der Abziehweg in der ursprünglichen Verbindungsrichtung im Operationsfeld versperrt oder erschwert ist.

Die Anwendung der Erfindung ist besonders vorteilhaft bei Schaftprothesen, und unter diesen wieder besonders vorteilhaft bei Hüftgelenk-Femurprothesen. Bei letzteren besteht nämlich die Möglichkeit, die Richtung des Instrumentenschafts etwa in Richtung des Prothesenschafts verlaufen zu lassen, wobei die für diese Stellung vorgesehen Arretierbohrung lateral vom Prothesenhals im Prothesenkragen bzw. im dortigen Schaftende leicht vorgesehen werden kann.

Die Erfindung wird im folgenden näher unter Bezugnahme auf die Zeichnung erläutert, die ein vorteilhaftes Ausführungsbeispiel veranschaulicht. Darin zeigen:

Fig. 1 eine Gesamtansicht der Prothesen/Instrumenten-Kombination im zusammengebauten Zustand etwas verkleinert und

Fig. 2 eine der Fig. 1 entsprechende Seitenansicht des Gabelendes des Instruments in etwa natürlicher Grösse.

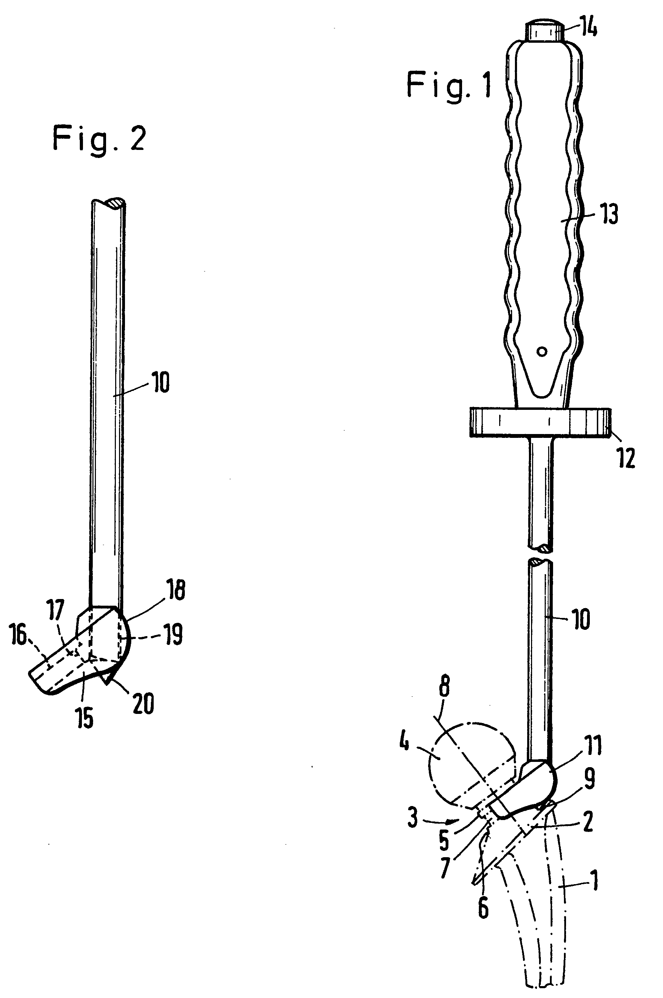

Die in Fig. 1 strichpunktiert angedeutete Prothese ist eine Hüftgelenk-Femurprothese mit Schaft 1, Kragen 2, Hals 3 und Gelenkkopf 4, der mit dem Hals einstückig verbunden oder über eine Konusverbindung aufgesetzt sein kann. Der Hals bildet zwischen einer Ringrippe 5 und einem den Kragen 2 oben abschliessenden Bund 6 eine Ringnut 7. Die Richtung 8 des Halses 3 ist gegenüber der Richtung des Schafts 1 in der bekannten Weise abgewinkelt.

Lateral vom Hals 3 enthält der Kragen 2 eine kegelige Bohrung 9 als Arretierbohrung, deren Lage und Achsrichtung etwa mit der Mittelachse des Schafts 1 übereinstimmt. Die laterale Begrenzungsfläche der Bohrung 9 schliesst mit der Schaftrichtung einen etwas keineren Winkel ein als die Rippen und Nuten 5 bis 7 am Prothesenhals.

Das Instrument besitzt einen Schaft 10, an dessen Ende die Gabel 11 sitzt. Am anderen Ende ist ein Teller 12 vorgesehen, dessen Unterfläche zur Beaufschlagung mittels eines Hammers beim Ausschlagen der Prothese vorgesehen ist. Es schliesst sich ein Handgriff 13 an, aus dem ein Vorsprung 14 des Schafts zur Bildung einer Schlagfläche beim Einschlagen einer Prothese herausragt. Das Instrument kann am Griff 13 leicht manipuliert werden, wobei auch Drehmomente zur Rotationslagekorrektur der Prothese während der Implantation aufgebracht werden können. Gewünschtenfalls kann der Griff auch quer zur Achsrichtung der Teile 10, 12, 14 vorgesehen werden, wenn die Aufbringung grösserer Drehmomente erwünscht ist.

Die Ebene der Gabel 11 steht quer zum Schaft 10, nämlich in einem solchen Neigungswinkel, der dem Winkel zwischen der Halsachse 8 und der Richtung des Schafts 1 der Prothese komplermentär ist. Dabei sind die Masse so eingerichtet, dass der Instrumentenschaft 10 etwa koaxial zum Prothesenschaft 1 liegt.

Die Gabel besitzt zwei Zinken 15, die in den Darstellungen hintereinander liegen und zwischen sich eine U-förmige Ausnehmung bilden, die im montierten Zustand den Hals 3 aufnimmt. Dabei weisen die einander zugewendeten Flächen der Zinken 15 in symmetrischer Anordnung eine Rippe 16 (strichpunktiert in Fig. 2) auf, deren Breite und Höhe derjenigen der Nut 7 am Hals der Prothese derart entspricht, dass die Rippe 16 in diese Nut eingreift, wenn das Instrument mit der Prothese verbunden ist. Die Rippe 16 kann auch in dem die Zinken 15 verbindenden Stegteil halbkreisförmig bei 17 durchgeführt sein, so dass sich ein maximaler Eingriff zwischen den Rippen und Nuten des Instruments und der Prothese erzielten lässt.

Der Stegteil 18 der Gabel 11 enthält eine Gewindebohrung 19, die das mit Gewinde versehene Ende des Instrumentenschafts 10 aufnimmt. Unterseitig ragt dieser mit der Konusspitze 20 als Arretierstift daraus hervor, deren Konuswinkel mit demjenigen der Arretierbohrung 9 übereinstimmt. Dies ist nicht unbedingt erforderlich, denn selbstverständlich wäre es auch denkbar, ein sphärisch ausgeführtes Ende des Arretierstifts mit einer kegeligen Arretierbohrung zusammenwirken zu lassen. Es könnten auch beide Teile angpassend zylindrisch ausgeführt sein.

Bei zurückgezogenem Arretierstift 10 kann die Gabel 11 des Instruments von jeder Seite her an den Prothesenhals angesetzt werden. Durch Drehung des Prothesenschafts kann der Arretierstift 20 dann in die Arretierbohrung 9 eingesenkt werden, wobei durch kräftiges Anziehen des Gewindes ein spielfreier Sitz erzielt wird, der auch ein feinfühliges Manipulieren der Prothese gestattet. Dabei sollen die medial zusammenwirkenden Flächen der Arretierbohrung und des Arretierstifts einen kleineren Winkel mit der Schaftlängsrichtung als die Rippen und Nuten von Prothesenhals und Gabel einschliessen, damit diese Teile nicht nur reibschlüssig sondern auch formschlüssig zusammengehalten sind. Dies ist insbesondere wichtig bei der Benutzung des Instruments zum Ausschlagen der Prothese, weil dann die Kräfte nur über die Gabel und die genannte laterale Fläche der Arretierelemente übertragen werden. Dies ist akzeptabel, weil beim Ausschlagen im allgemeinen geringere Kräfte als beim Einschlagen angewendet werden. Die Einschlagkräfte werden hingegen von dem Instrumentenschaft 10 geradlinig ohne den Umweg über die Gabel 11 von dem Arretierstift 20 auf die Arretierbohrung 9 und damit auf den Prothesenschaft übertragen.

## Patentansprüche

1. Gelenkendoprothese und Instrument zum Ein- bzw. Ausschlagen derselben, die zur gegenseitigen Verbindung mit zusammenwirkenden Rippen (5, 6) und Nuten (7) einerseits in einer am Ende des Instrumentenschafts (10) angeordneten Gabel (11) und andererseits am Hals (3) der Prothese vorgesehen sind, wobei die Richtung des Instrumentenschafts (10) quer zur Gabelebene verläuft, dadurch gekennzeichnet, dass das Ende des Instrumentenschafts (10) als Arretierstift (20) durch eine Bohrung (19) der Gabel (11) hindurchragt und die Prothese eine Arretierungsausnehmung (9) zur Aufnahme des Arretierstifts (20) aufweist.

2. Prothese und Instrument nach Anspruch 1, dadurch gekennzeichnet, dass die Rippe (5, 6) bzw. Nut (7) am Preothesenhals (3) ringförmig ausgebildet ist (sind).

3. Prothese und Instrument nach Anspruch 1 oder 2, dadurch gekennzeichnet, dass die Arretierausnehmung (9) eine Arretierbohrung ist.

4. Prothese und Instrument nach Anspruch 3, dadurch gekennzeichnet, dass der Arretierstift (20) und die Arretierbohrung (9) über wenigstens eine quer zur Schaftrichtung verlaufende Fläche zusammenwirken.

5. Prothese und Instrument nach einem der Ansprüche 1 bis 4, dadurch gekennzeichnet, dass der Schaft (10) und die ihn in der Gabel (11) aufnehmende Bohrung (19) mit zusammenwirkendem Gewinde versehen sind.

6. Prothese und Instrument nach einem der Ansprüche 1 bis 5, dadurch gekennzeichnet, dass mehrere Arretiervertiefungen (9) am Umfang vorgesehen sind.

## Claims

1. A joint endoprosthesis and an instrument for driving it in and/or driving it out, which for mutual connection are provided with cooperating ribs (5, 6) and grooves (77 on the one hand, in a fork (11) disposed at the end of the instrument shank (10) and, on the other hand, on the neck (3) of the prosthesis, wherein the direction of the instrument shank (10) extends transversely to the end of the fork, characterised in that the end of the instrument shank (10) projects, as a locating pin (20), through a bore (19) in the fork (11) and the prosthesis has a locating recess (9) to accomodate the locating pin (20).

2. A prosthesis and an instrument according to Claim 1, characterised in that the rib (5, 6) and/or groove (77 is/are formed annulary on the prosthesis neck (3).

3. A prosthesis and an instrument according to Claim 1 or 2, characterised in that the locating recess (9) is a locating bore.

4. A prosthesis and an instrument according to Claim 3, characterised in that the locating pin (20) and the locating bore (9) cooperate by means of at least one surface extending transversely to the shank direction.

5. A prosthesis and an instrument according to any one of Claims 1 to 4, characterised in that the shank (10) and the bore (19) accomodating it in the fork (11) are provided with cooperating threads.

6. A prosthesis and an instrument according to any one of Claims 1 to 5, characterised in that a plurality of locating depressions (9) are provided over the periphery.

**Revendications**

1. Endoprothèse d'articulation et appareil pour l'enfoncer ou l'extraire, qui sont munis, pour une liaison mutuelle, de nervures (5, 6) et rainures (7) coopérantes formées d'une part dans une fourchette (11) disposée à une extrémité de la tige d'appareil (10) et d'autre part sur le col (3) de la prothèse, la direction de la tige d'appareil (10) étant orientée perpendiculairement au plan de la fourchette, caractérisés en ce que l'extrémité de la tige d'appareil (10) traverse, comme une broche d'arrêt (20), un trou (19) de la fourchette (11) et la prothèse comporte un évidement d'arrêt (9) servant à recevoir la broche d'arrêt (20).

2. Prothèse et instrument selon la revendication 1, caractérisé en ce que la nervure (5, 6) ou la rainure (7) prévue sur le col de prothèse (3) est réalisée avec une forme annulaire.

3. Prothèse et instrument selon la revendication 1 ou 2, caractérisés en ce que l'évidement d'arrêt (9) est un trou d'arrêt.

4. Prothèse et instrument selon la revendication 3, caractérisé en ce que la broche d'arrêt (20) et le trou d'arrêt (9) coopèrent par l'intermédiaire d'au moins une surface orientée perpendiculairement à la direction de tige.

5. Prothèse et instrument selon une des revendications 1 à 4, caractérisés en ce que la tige (10) et le trou (19) la recevant dans la fourchette (11) sont pourvus de filetages coopérants.

6. Prothèse et instrument selon une des revendications 1 à 5, caractérisés en ce que plusieurs creux d'arrêt (9) sont prévus sur la périphérie.

Fig. 2

Fig. 1